# EUROPEAN PATENT APPLICATION

(11) **EP 3 708 180 A1**
(43) Date of publication of application: **16.09.2020**
(21) Application number: 19163295.9
(22) Date of filing: 15.03.2019
(51) Int. Cl.: A61K 38/16, A61K 35/741, C07K 14/435

(54) **TRANSGENIC PROBIOTIC E. COLI CELL**

(71) Applicant: Freie Universität Berlin, 14195 Berlin (DE)
(72) Inventor: Tedin, Lydia, 14532 Kleinmachnow (DE); Tedin, Karsten, 14532 Kleinmachnow (DE); Hartmann, Susanne, 12307 Berlin (DE)
(74) Representative: Maikowski & Ninnemann Patentanwälte Partnerschaft mbB

(57) **Abstract**

The invention relates to a transgenic probiotic *E. coli* cell genetically engineered to express and secrete a nematode-derived immunomodulator. The cell comprises chromosomal *E. coli* DNA, wherein the genetic information for expression and secretion of the nematode-derived immunomodulator is integrated into the chromosomal *E. coli* DNA and wherein the *E. coli cell* is an auxotrophic cell lacking genetic information necessary for synthesizing a compound required for the growth of the *E. coli cell.*

## Description

The present invention relates to a transgenic probiotic *E*. *coli cell* according to the preamble of claim 1, to a pharmaceutical composition comprising such an *E. coli* cell according to the preamble of claim 9, to further medical uses of such a transgenic probiotic *E. coli cell* or such pharmaceutical composition according to the preambles of claims 10, 11 and 12, as well as to a method for manufacturing such a transgenic probiotic *E. coli cell* according to the preamble of claim 14.

EP 2 056 867 B1 describes cystatin from parasitic nematodes and its suitability for the treatment of autoimmune or allergic diseases. This cystatin is a protein which is secreted from parasitic nematodes into the host and changes an immune response of the host into an anti-inflammatory response.

EP 2 892 917 B1 describes a transgenic probiotic microorganism that is genetically engineered to express and secrete nematode-derived cystatin. The genetic information for expressing and secreting the nematode-derived cystatin is present on a plasmid. This patent further describes to use such a transgenic probiotic microorganism as medicament against chronic inflammatory gastrointestinal diseases.

Although the transgenic probiotic *E. coli* bacteria secreting cystatin known from prior art have been shown to have good efficacy, there exist safety concerns regarding an approval as a drug. This is due to the fact that the transgenic *E*. *coli* strain that has been used so far carries the genetic information for cystatin production on a plasmid. In principle, it cannot be ruled out that the *E. coli* cells may pass on the genetic information on cystatin production to pathogenic microorganisms. In addition, currently there exists no possibility of regulating the activity of the *E. coli* cells in a patient. Specifically, it is not possible to discontinue the medication in a controlled manner within a short time.

It is an object of the present invention to provide a novel transgenic probiotic microorganism that overcomes the disadvantages of prior art.

This object is achieved with a transgenic probiotic *E. coli cell* having the features of claim 1.

Such an *E. coli* cell is genetically engineered to express and secrete a nematode-derived immunomodulator. Thereby, the *E. coli* cell comprises - as all common *E. coli* cells do - chromosomal *E. coli* DNA.

According to the claimed invention, the genetic information for expression and secretion of the nematode-derived immunomodulator is integrated into the chromosomal *E. coli* DNA. Furthermore, the *E. coli* cell is modified such to be an auxotrophic cell. Thus, the *E. coli cell* lacks genetic information which is necessary for synthesizing a compound that is required for the growth of the *E. coli* cell. Thereby, "lacking genetic information" includes "lacking functioning genetic information". Thus, by mutating genetic information coding for an essential protein by deletion or insertion of individual bases, the proper function of the according gene can be destroyed. Then, the *E. coli* cell lacks functioning genetic information for expressing an essential protein.

By genetically modifying the *E. coli* cell such that it is an auxotrophic cell and that it comprises the genetic information for expression and secretion of the nematode-derived immunomodulator, it is possible that the *E. coli* cell expresses the nematode-derived immunomodulator but also fulfils safety criteria for an application as drug.

Furthermore, due to the integration of the genetic information (e.g., DNA) for expression and secretion of the nematode-derived immunomodulator into the chromosomal *E. coli* DNA, the risk of a horizontal genetic transfer is significantly reduced or fully avoided. Such horizontal genetic transfer is in principle possible if genetic information is placed on a plasmid being present in a microorganism. Thus, the presently claimed transgenic probiotic *E*. *coli cell* has the following properties:
- Auxotrophy: Targeted gene deletion prevents proliferation of the transgenic strain when it enters the environment with the feces. The bacterium lacks the gene sequence to produce a vital (essential) substance. Thus, it is dependent on a supplementation with the vital substance.
- Controllability: When the bacterium is supplemented with the vital substance (e.g., thymidine in case of a deletion of the thymidylate synthase gene), the bacterium is capable of growing and expressing proteins, antigens and/or other factors. In this way, the vitality and activity of the bacterium in the patient's body can be regulated, and the future medication can be discontinued if the intake of the vital substance is stopped. Thus, the probiotic *E. coli* cell fulfils safety criteria of an application as drug and thus can be approved as a drug. The vital substance can be administered to a patient in form of a food supplement. Then, this food supplement is necessary to maintain the probiotic. Discontinuance guarantees a rapid disappearance of the medication due to the loss of the bacterium.
- Stable integration of the genetic information on the immunomodulator into the chromosome of the probiotic cell: The genomic integration of the nematode-derived immunomodulator prevents the possibility of exchange of the immunomodulator sequence with other bacteria of intestinal microbiota. The so-called horizontal gene transfer is thus prevented.

In an embodiment, the *E. coli cell* is a cell of the strain *E. coli* Nissle 1917. This *E. coli* strain has already been used in prior art as probiotic *E. coli* strain. The (genetically non-modified) strain *E. coli* Nissle 1917 is approved as medicament under the trademark Mutaflor. *E. coli* Nissle represents an excellent vehicle for nematode-derived immunomodulators. Originally isolated from humans, *E. coli* Nissle temporarily colonizes the human and porcine intestine and shows positive effects in clinical colitis studies. *E. coli* Nissle leads to the strengthening of intestinal barrier function.

In an embodiment, the nematode-derived immunomodulator belongs to the group of excretory-secretory (E-S) products, in particular to the group of E-S proteins.

In an embodiment, the nematode-derived immunomodulator is a cystatin. In an embodiment, the nematode-derived immunomodulator is a chitinase. In an embodiment, the nematode-derived immunomodulator is a cystatin or a chitinase.

In an embodiment, the nematode-derived immunomodulator comprises or consists of an amino acid sequence according to SEQ ID NO. 1; or an amino acid sequence that is at least 70 % identical, in particular at least 80 % identical, in particular at least 90 % identical, in particular at least 99 % identical to an amino acid sequence according to SEQ ID NO. 1.

SEQ ID NO. 1 represents the amino acid sequence of cystatin derived from *Acanthocheilonema viteae* (also denoted as Av cystatin). It can be accessed in gene bank of the National Center for Biotechnology Information (NCBI) (www.ncbi.nlm.nih.gov) with the accession numbers L43053 (underlying gene) or AAA87228 (protein).

In an embodiment, the nematode-derived immunomodulator comprises or consists of an amino acid sequence according to SEQ ID NO. 2 or; or an amino acid sequence that is at least 70 % identical, in particular at least 80 % identical, in particular at least 90 % identical, in particular at least 99 % identical to an amino acid sequence according to SEQ ID NO. 2.

SEQ ID NO. 2 represents the amino acid sequence of cystatin derived from *Onchocerca volvulus* (also denoted as Ov cystatin). It can be accessed in NCBI's gene bank with the accession number M37105 (underlying gene) or AAA29423 (protein).

In an embodiment, the nematode-derived immunomodulator comprises or consists of an amino acid sequence according to SEQ ID NO. 3; or an amino acid sequence that is at least 70 % identical, in particular at least 80 % identical, in particular at least 90 % identical, in particular at least 99 % identical to an amino acid sequence according to SEQ ID NO. 3.

SEQ ID NO. 3 represents the amino acid sequence of cystatin derived from *Brugia malayi* (also denoted as Bm cystatin). It can be accessed in NCBI's gene bank with the accession number AF177193 (underlying gene) or AAD51086 (protein).

In an embodiment, the nematode-derived immunomodulator comprises or consists of an amino acid sequence according to SEQ ID NO. 4; or an amino acid sequence that is at least 70 % identical, in particular at least 80 % identical, in particular at least 90 % identical, in particular at least 99 % identical to an amino acid sequence according to SEQ ID NO. 4.

SEQ ID NO. 4 represents the amino acid sequence of an acidic mammalian chitinase derived from the pig whipworm *Trichuris suis* (also denoted as TsES1). This chitinase is also a very potent immunomodulator that belongs to the excretory/secretory nematode proteins. It can be accessed in NCBI's gene bank with the accession number KL363292 (underlying gene) or KFD48490 (protein).

Generally, all genes that have described in the literature to be essential for *E*. *coli* cells, in particular for the strain *E. coli* Nissle 1917, can be subject to a deletion or modification in order to produce an auxotrophic *E. coli* cell. In an embodiment, the lacking genetic information necessary for synthesizing a compound required for the growth of the *E. coli* cell is chosen from the group consisting of genetic information coding for a component of p-aminobenzoate synthase (*pabA, pabB,* and/or *pabC*), genetic information coding for quinolinate synthase A (*nadA*), genetic information coding for L-aspartate oxidase (*nadB*), genetic information coding for 5-enolpyruvylshikimate-3-phosphate synthetase (*aroA*), genetic information coding for adenylosuccinate synthetase (*purA*), and genetic information coding for thymidylate synthase (*thyA*). In an embodiment, the *E. coli cell* is auxotrophic due to a lacking or defect *thyA* gene.

A deletion or a mutation of a gene encoding a component of p-aminobenzoate synthase (*pabA, pabB,* and/or *pabC*) induces a dependency of the *E. coli cell* on p-aminobenzoate (pABA). A deletion or a mutation of a gene encoding quinolinate synthase A (*nadA*) or L-aspartate oxidase (*nadB*) induces a dependency of the *E. coli cell* for exogenous nicotinamide adenine dinucleotide (NAD) since these genes are essential for a de novo biosynthesis of NAD. A deletion or a mutation of a gene encoding 5-enolpyruvylshikimate-3-phosphate synthetase (*aroA*) induces a dependency of the *E. coli* cell on 5-enolpyruvylshikimate-3-phosphate. A deletion or a mutation of a gene encoding adenylosuccinate synthetase (*purA*) induces a dependency of the *E. coli* cell on adenine. A deletion or a mutation of a gene encoding thymidylate synthase (*thyA*) induces a dependency of the *E. coli cell* on thymine or thymidine.

In addition to the lacking possibility of discontinuing the use of probiotic *E. coli* cells known from prior art as a drug, another decisive disadvantage exists in prior art. Probiotic *E. coli* cells known from prior art cannot be detected or quantified from the patient's feces using classical microbiological methods. Since these bacteria were originally isolated from a human being, it can be assumed that it is basically capable of colonizing the human intestine at least temporarily. However, depending on dietary habits, health status, medication intake and genetic differences between patients, it can be assumed that the colonization period of the probiotic in the intestine differs from patient to patient. The amount of a (pharmaceutical) substance produced by the probiotic bacteria after their ingestion is therefore also individually different (even if each patient takes the same dose of the probiotic). Therefore, an individual optimal dosage of the probiotic is difficult to achieve under the current conditions. This is a major pharmacological shortcoming of the previous approach.

In an embodiment, the transgenic probiotic *E. coli cell* comprises genetic information coding for a marker substance. In an embodiment, this genetic information is also integrated into the chromosomal *E. coli* DNA. The marker substance or marker protein is a protein that usually does not occur in the gastrointestinal tract of a human or an animal. Thus, the marker protein is not synthesized by enterobacteria that are normally present in the gastrointestinal tract of a human or animal. Rather, the marker protein is a protein which is exclusively synthesized in the gastrointestinal tract of human or animal by the transgenic bacteria if these are present in the gastrointestinal tract. Furthermore, the marker protein is a protein which is harmless to a human or animal.

In an embodiment, this genetic information is coding for a marker protein that is neither excreted by the human microbiota nor by human intestinal cells, and that does not usually occur in plant or animal foods, or that can be easily and safely avoided to be ingested with food.

In an embodiment, the marker substance and the pharmaceutically active substance (i.e., the nematode-derived immunomodulator) are always produced and secreted in a strongly correlating manner in the genetically modified probiotic *E. coli* cell so that the detection of the marker substance enables direct conclusions to be drawn about the production of the pharmaceutically active substance in the patient's intestine. Such equal production of the nematode-derived immunomodulator and the marker protein can be particularly easy achieved if the genetic information responsible for expressing (and secreting) the immunomodulator and the marker protein are located between the same promoter and the same stop codon. In such a case, they will be always expressed uniformly. In an embodiment, the marker substance and the pharmaceutically active substance are produced in equal proportions.

In an embodiment, the marker protein secreted by the genetically modified probiotic is detected in the patient's feces, e.g., via a chromatographic immunoassay. A quick test may be used for this. Such quick test may be applied in a domestic environment. With the help of several or regular test results, a long-term dosage schedule can be determined individually for each patient under medical supervision.

In an embodiment, the marker substance is a cardiac troponin. Cardiac troponins, such as cardiac troponin I (c-troponin I) or cardiac troponin T (c-troponin T), fulfil all requirements listed above. They are harmless to a human or animal and are not usually produced by enterobacteria within the gastrointestinal tract of a human or animal. Rather, they are exclusively expressed in the heart muscle and thus do not usually occur in the intestine or the feces of a human or an animal.

In an embodiment, the marker protein is cardiac troponin T. Cardiac troponin T occurs in various isoforms. In an embodiment, the cardiac troponin T used as a marker substance comprises or consists of an amino acid sequence according to SEQ ID NO. 5; or an amino acid sequence that is at least 70 % identical, in particular at least 80 % identical, in particular at least 90 % identical, in particular at least 99 % identical to an amino acid sequence according to SEQ ID NO. 5.

SEQ ID NO. 5 represents the amino acid consensus sequence of c-troponin T derived from humans. One of its underlying isoforms, isoform 1, can be accessed in NCBI's gene bank with the accession number NP_000355 (protein).

In an embodiment, the marker protein is cardiac troponin I. In an embodiment, the cardiac troponin I used as a marker substance comprises or consists of an amino acid sequence according to SEQ ID NO. 6; or an amino acid sequence that is at least 70 % identical, in particular at least 80 % identical, in particular at least 90 % identical, in particular at least 99 % identical to an amino acid sequence according to SEQ ID NO. 6.

SEQ ID NO. 6 represents the amino acid sequence of c-troponin I derived from humans. It can be accessed in NCBI's gene bank with the accession number NP_000354 (protein).

In an embodiment, the genetic information for expression and secretion of the nematode-derived immunomodulator replaces the genetic information that was deleted from the *E. coli* cell in order to make the strain auxotrophic. Then, the genetic information for expression and secretion of the immunomodulator is integrated into the chromosomal *E. coli* DNA at a site at which the genetic information necessary for synthesizing a compound required for the growth of the *E. coli* cell, which is lacking, has been located prior to its deletion. Such a replacement can be achieved in a particularly appropriate manner by genetic exchange, e.g. by applying the lambda Red recombination technique.

An aspect of the present invention relates to a pharmaceutical composition comprising a transgenic probiotic *E. coli* cell according to the preceding explanations and at least one pharmaceutically acceptable excipient. Such a pharmaceutical composition can be ingested by the patient in need thereof. Then, the probiotic transgenic *E. coli* cell will express and secrete the nematode-derived immunomodulator into the gastrointestinal tract of the patient. There, it will be active in immunomodulatory way and will change an immune response of the patient to an anti-inflammatory reaction.

An aspect of the invention relates to a novel medical use of the transgenic probiotic *E. coli cell* according to the preceding explanations or the pharmaceutical composition according to the preceding explanations, namely for the treatment of allergic rhinitis and/or allergic airway hyperreactivity, in particular an allergic hyperreactivity in the lung. Allergic airway hyperreactivity, which is sometimes also referred to as asthma, may by induced by a non-treated allergic rhinitis or an extended exposure of a patient to allergens. It could be shown that treating a patient suffering from allergic rhinitis and/or allergic airway hyperreactivity by ingestion of a transgenic *E. coli* cell significantly ameliorates the health status of the patient even though the transgenic *E*. *coli* cells never reach the airways of the patient. However, the immunomodulator expressed and secreted into the patient's intestines by the transgenic *E*. *coli cell* is capable of curing allergy-related conditions of the patient's airways.

Another aspect of the invention relates to another novel medical use of the transgenic probiotic *E. coli* cell according to the preceding explanations or the pharmaceutical composition according to the preceding explanations, namely for the treatment of a gastro-intestinal inflammatory disease and/or an inflammatory bowel disease.

In an embodiment, the inflammatory bowel disease is Crohn's disease and/or ulcerative colitis. The transgenic *E. coli* Nissle cells, producing and releasing Av cystatin, show a significant positive impact on colitis. In a murine DSS-induced colitis model, oral administration of these transgenic *E*. *coli* Nissle cells significantly improved the clinical picture. This improvement is associated with a reduced migration of inflammatory monocytes/macrophages to the intestine, a suppressed production of chemokines, a dampened immune cell migration to the gut and a reduced expression of pro-inflammatory cytokines (IL-17, IL-6). Importantly, it could already be shown that these transgenic *E*. *coli* Nissle cells colonized the human-like porcine gut and led to a significant reduction of inflammation in a piglet model.

In an embodiment, the transgenic probiotic *E. coli* cell or the pharmaceutical composition is used to treat patients having an impaired intestinal barrier function, an impaired intestinal transepithelial resistance and/or an intestinal hyperpermeability.

In an aspect, the present invention relates to medical method for treating a gastro-intestinal inflammatory disease and/or an inflammatory bowel disease of the patient by administering a pharmaceutically active amount of probiotic transgenic *E. coli* cells comprising chromosomal *E. coli* DNA to a patient, wherein the *E. coli* cells are genetically engineered to express and secrete a nematode-derived immunomodulator, wherein the genetic information for expression and secretion of the nematode-derived immunomodulator is integrated into the chromosomal *E. coli* DNA and wherein the *E. coli* cells are auxotrophic cells lacking genetic information necessary for synthesizing a compound required for the growth of the *E. coli* cells.

In an aspect, the present invention also relates to a method for manufacturing a transgenic probiotic *E. coli* cell according to the preceding explanations. Thereby, this manufacturing method comprises the steps explained in the following.

First, a probiotic *E. coli cell* having chromosomal *E. coli* DNA is provided.

Then, genetic information necessary for synthesizing a compound required for the growth of the *E. coli* cell is excised from the chromosomal *E. coli* DNA and replaced with genetic information for expression and secretion of a nematode-derived immunomodulator, and integrated into the chromosomal *E. coli* DNA.

This method can be carried out, e.g., by applying the bacteriophage lambda Red recombinase technique. With this technique, it is possible to exchange genetic information present in the chromosomal *E. coli* DNA with external genetic information (in the present case with genetic information encoding a nematode-derived immunomodulator).

In an embodiment, the genetic information for expression and secretion of a nematode-derived immunomodulator is integrated into the chromosomal *E. coli* DNA at the site at which the genetic information necessary for synthesizing a compound required for the growth of the *E*. *coli* cell was excised. Thus, a gene exchange is carried out in this embodiment.

In an embodiment, the method is carried out by including integration of an antibiotic resistance gene during genetic engineering. This simplifies selection of bacteria that have lost the genetic information to be excised and that have received the genetic information to be integrated into the chromosomal DNA by applying selection pressure on the basis of an antibiotic. Once the chromosomal integration has been verified using either the polymerase chain reaction (PCR) with appropriate oligonucleotides flanking the integration site in the chromosome or by DNA sequencing of the region where the integration has taken place, the antibiotic resistance gene can be removed. This is done by providing a temporary source of flippase (also known as FLP recombinase) expressed from a plasmid, which can later be eliminated by growth at 37 °C.

All embodiments of the described transgenic probiotic *E. coli* cell can be combined in any desired way and can be transferred to the pharmaceutical composition and to the medical uses of the transgenic probiotic *E. coli* cell and/or the pharmaceutical composition, and vice versa. Furthermore, the various embodiments of the *E. coli* cell and their combinations can be transferred to the method for manufacturing such an *E. coli* cell, and vice versa. Additionally, all embodiment of the method for manufacturing the *E. coli* cell and their combinations can be transferred to the medical uses of the transgenic probiotic *E. coli* cell and/or the pharmaceutical composition, and vice versa.

Further details of aspects of the present invention will be explained with respect to an exemplary embodiment and a Figure.

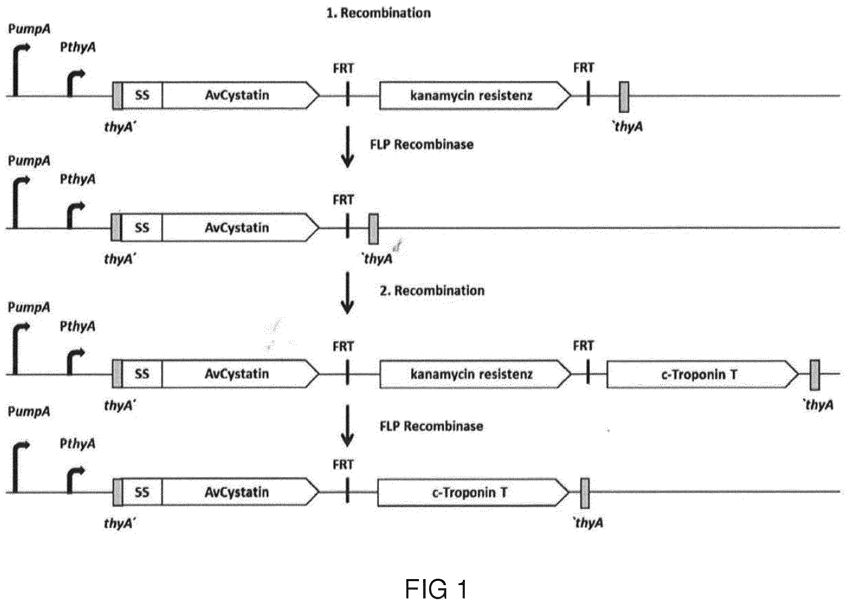

- Figure 1: shows a schematic sketch on the gene exchange method to be accomplished for manufacturing a transgenic probiotic *E. coli* cell.

In a first recombination step, a gene encoding thymidylate synthetase (*thyA*), which is an essential gene for *E. coli,* is exchanged by the bacteriophage lambda Red recombinase technique for a gene encoding Av cystatin (SEQ ID NO. 1). Thereby, this gene is inserted into the chromosomal *E. coli* DNA together with a gene encoding kanamycin resistance and which is flanked by flippase recognition targets (FRT). The gene encoding Av cystatin also comprises a signal sequence (SS) and is under the control of the promoter of *umpA* (another essential *E*. *coli* gene) and the promotor of *thyA.* Since both the *umpA* and the *thyA* gene are essential genes, the control of these promoters ensures that the newly introduced genetic information is indeed expressed.

The *E. coli* cells are then subjected to kanamycin treatment. Cells, in which the gene insertion has been successful, will survive this treatment due to the introduced kanamycin resistance. All other cells will be destroyed by kanamycin.

Afterwards, flippase (FLP) is added to the *E. coli* cells. Flippase is a recombinase and recognizes flippase recognition targets (FRT). It can be used for an excision of genetic information located between two FRTs. In doing so, the genetic information encoding kanamycin resistance can be excised. One FRT remains in the chromosomal *E. coli* DNA so that another FLP recombination step is possible.

This second recombination serves for introducing a combined genetic sequence encoding kanamycin resistance and encoding c-troponin T (SEQ ID NO. 5) into the chromosomal *E. coli* DNA. C-troponin T serves as marker substance.

Afterwards, the *E. coli* cells are subjected again to kanamycin treatment. Cells, in which the gene insertion has been successful, will survive this treatment due to the introduced kanamycin resistance. All other cells will be destroyed by the kanamycin.

Then, FLP recombinase can be once again added to the *E. coli* cells, leading to an excision of the kanamycin resistance. The resulting *E. coli* cells will only comprise genetic information for expressing Av cystatin (including a signal sequence for secreting the Av cystatin) and for expressing c-troponin T. Thereby, the expression of both Av cystatin and c-troponin T is under the control of the *umpA* and the *thyA* promoters. Consequently, there is a strong correlation between expression of Av cystatin and c-troponin T. Thus, by quantifying the amount of produced c-troponin T, a direct measure for the produced amount of Av cystatin exists. In an embodiment, the same amount of Av cystatin and c-troponin T is produced.

The final transgenic *E. coli* cell does not comprise a kanamycin resistance. Thus, no spread of antibiotic-resistant *E. coli* cells needs to be feared. Since the transgenic *E. coli* cells cannot synthesize thymidine, it is necessary to supplement the culture of those *E. coli* cells with thymidine or thymine. Without such supplementation, the *E. coli* cells rapidly die and will be automatically excreted by the patient since they can no longer survive in the body of the patient.

Since the genetic information encoding Av cystatin and c-troponin T is integrated into the chromosome of the *E. coli* cells, no loss of this genetic information due to plasmid loss needs to be feared. Such plasmid loss can occur in case of *E. coli* cells harboring a plasmid when selection pressure is removed. Furthermore, a sequence exchange between different *E. coli* cells due to plasmid exchange can occur in those cells.

The used recombination method (making use of a bacteriophage lambda Red recombinase) as well as the use of template constructs enable an exchange of the signal sequence used for secretion of Av cystatin by another signal sequence to ameliorate the secretion of Av cystatin, if necessary.

Due to the c-troponin T marker (or, alternatively, other comparable markers) it is possible to monitor the viability and productivity of the genetically engineered *E. coli* cells and to optimize the frequency of medication in individual patients.

## Claims

1. Transgenic probiotic *E. coli* cell genetically engineered to express and secrete at least one nematode-derived immunomodulator, comprising chromosomal *E. coli* DNA,
**characterized**
**in that** the genetic information for expression and secretion of the nematode-derived immunomodulator is integrated into the chromosomal *E. coli* DNA and in that the *E. coli* cell is an auxotrophic cell lacking genetic information necessary for synthesizing a compound required for the growth of the *E. coli* cell.

2. Transgenic probiotic *E. coli cell* according to claim 1, **characterized in that** the *E. coli* cell is a cell of the strain *E. coli* Nissle 1917.

3. Transgenic probiotic *E. coli* cell according to claim 1 or 2, **characterized in that** the nematode-derived immunomodulator comprises or consists of an amino acid sequence according to SEQ ID NO. 1, 2, 3 or 4; or an amino acid sequence that is at least 70 % identical, in particular at least 80 % identical, in particular at least 90 % identical, in particular at least 99 % identical to an amino acid sequence according to SEQ ID NO. 1, 2, 3 or 4.

4. Transgenic probiotic *E. coli cell* according to any of the preceding claims, **characterized in that** the lacking genetic information necessary for synthesizing a compound required for the growth of the *E. coli* cell is chosen from the group consisting of genetic information coding for components of p-aminobenzoate synthase, genetic information coding for quinolinate synthase A, genetic information coding for L-aspartate oxidase, genetic information coding for 5-enolpyruvylshikimate-3-phosphate synthetase, genetic information coding for adenylosuccinate synthetase, and genetic information coding for thymidylate synthase.

5. Transgenic probiotic *E. coli cell* according to any of the preceding claims, **characterized in that** it comprises genetic information coding for a marker substance that is integrated into the chromosomal *E. coli* DNA.

6. Transgenic probiotic *E. coli* cell according to claim 5, **characterized in that** the marker substance is chosen from the group consisting of cardiac troponins.

7. Transgenic probiotic *E. coli* cell according to claim 5 or 6, **characterized in that** the marker substance is troponin T.

8. Transgenic probiotic *E. coli cell* according to any of the preceding claims, **characterized in that** the genetic information for expression and secretion of the nematode-derived immunomodulator is integrated into the chromosomal *E. coli* DNA at a site at which the genetic information necessary for synthesizing a compound required for the growth of the *E. coli* cell, which is lacking, has been located prior to its deletion.

9. Pharmaceutical composition comprising a transgenic probiotic *E. coli* cell according to any of the preceding claims and at least one pharmaceutically acceptable excipient.

10. Transgenic probiotic *E. coli* cell according to any of claims 1 to 8 or pharmaceutical composition according to claim 9 for use in the treatment of allergic rhinitis or allergic airway hyperreactivity.

11. Transgenic probiotic *E. coli* cell according to any of claims 1 to 8 or pharmaceutical composition according to claim 9 for use in the treatment of a gastro-intestinal inflammatory disease or of an inflammatory bowel disease.

12. Transgenic probiotic *E. coli* cell according to any of claims 1 to 8 or pharmaceutical composition according to claim 9 for use in the treatment of Crohn's disease and/or ulcerative colitis.

13. Transgenic probiotic *E. coli* cell according to any of claims 1 to 8 or pharmaceutical composition according to claim 9 for use according to any of claims 10 to 12, wherein patients having impaired intestinal barrier function, impaired intestinal transepithelial resistance and/or intestinal hyperpermeability are treated.

14. Method for manufacturing a transgenic probiotic *E. coli* cell according to any of claims 1 to 8, comprising the following steps:
a) providing a probiotic *E. coli* cell having chromosomal *E. coli* DNA,
b) excising genetic information necessary for synthesizing a compound required for the growth of the *E. coli* cell from the chromosomal *E. coli* DNA,
c) integrating genetic information for expression and secretion of at least one nematode-derived immunomodulator into the chromosomal *E. coli* DNA.

15. Method according to claim 14, **characterized in that** the genetic information for expression and secretion of the at least one nematode-derived immunomodulator is integrated into the chromosomal *E. coli* DNA at the site at which the genetic information necessary for synthesizing a compound required for the growth of the *E. coli* cell was excised.


SEQUENCE LISTING

<110>  Freie Universität Berlin

<120>  Transgenic probiotic E. coli cell

<130>  FU202EP

<160>  6

<170>  PatentIn version 3.5

<210>  1
<211>  157
<212>  PRT
<213>  Acanthocheilonema viteae

<400>  1

Met Met Leu Ser Ile Lys Glu Asp Gly Leu Leu Val Val Leu Leu Leu
1               5                   10                  15

Ser Phe Gly Val Thr Thr Val Leu Val Arg Cys Glu Glu Pro Ala Asn
            20                  25                  30

Met Glu Ser Glu Val Gln Ala Pro Asn Leu Leu Gly Gly Trp Gln Glu
        35                  40                  45

Arg Asn Pro Glu Glu Lys Glu Ile Gln Asp Leu Leu Pro Lys Val Leu
    50                  55                  60

Ile Lys Leu Asn Gln Leu Ser Asn Val Glu Tyr His Leu Met Pro Ile
65                  70                  75                  80

Lys Leu Leu Lys Val Ser Ser Gln Val Val Ala Gly Leu Arg Tyr Lys
                85                  90                  95

Met Glu Ile Gln Val Ala Gln Ser Glu Cys Lys Lys Ser Ser Gly Glu
            100                 105                 110

Glu Val Asn Leu Lys Thr Cys Lys Arg Leu Glu Gly His Pro Asp Gln
        115                 120                 125

Ile Ile Thr Leu Glu Ala Trp Glu Lys Ser Trp Glu Asn Phe Leu Gln
    130                 135                 140

Val Lys Ile Leu Glu Lys Lys Glu Val Leu Ser Ser Val
145                 150                 155

<210>  2
<211>  162
<212>  PRT
<213>  Onchocerca volvulus

<400>  2

Met Leu Thr Ile Lys Asp Gly Thr Leu Leu Ile His Leu Leu Leu Phe
1               5                   10                  15

Ser Val Val Ala Leu Val Gln Leu Gln Gly Ala Lys Ser Ala Arg Ala
            20                  25                  30

Lys Asn Pro Ser Lys Met Glu Ser Lys Thr Gly Glu Asn Gln Asp Arg
        35                  40                  45

Pro Val Leu Leu Gly Gly Trp Glu Asp Arg Asp Pro Lys Asp Glu Glu
    50                  55                  60

Ile Leu Glu Leu Leu Pro Ser Ile Leu Met Lys Val Asn Glu Gln Ser
65                  70                  75                  80

Asn Asp Glu Tyr His Leu Met Pro Ile Lys Leu Leu Lys Val Ser Ser
                85                  90                  95

Gln Val Val Ala Gly Val Lys Tyr Lys Met Asp Val Gln Val Ala Arg
            100                 105                 110

Ser Gln Cys Lys Lys Ser Ser Asn Glu Lys Val Asp Leu Thr Lys Cys
        115                 120                 125

Lys Lys Leu Glu Gly His Pro Glu Lys Val Met Thr Leu Glu Val Trp
    130                 135                 140

Glu Lys Pro Trp Glu Asn Phe Met Arg Val Glu Ile Leu Gly Thr Lys
145                 150                 155                 160

Glu Val



<210>  3
<211>  161
<212>  PRT
<213>  Brugia malayi

<400>  3

Met Met Ser Thr Met Ser Ile Lys Glu Gly Leu Leu Val Ile Leu Leu
1               5                   10                  15

Ser Leu Phe Leu Phe Asp Thr Thr Ala Leu Ile His Arg Arg Glu Ile
            20                  25                  30

Pro His Met Glu Ser Lys Gly Gln Met Gln Arg Gly Gln Val Leu Leu

        35                  40                  45

Gly Gly Trp Gln Glu Arg Ser Pro Glu Asp Asn Glu Ile Leu Glu Leu
    50                  55                  60

Leu Pro Ser Val Leu Thr Lys Val Asn Gln Gln Ser Asn Asp Glu Tyr
65                  70                  75                  80

His Leu Met Pro Ile Lys Leu Leu Lys Val Ser Ser Gln Val Val Ala
                85                  90                  95

Gly Val Lys Tyr Lys Met Glu Val Gln Val Ala Arg Ser Glu Cys Lys
            100                 105                 110

Lys Ser Ala Ser Glu Gln Val Asn Leu Lys Thr Cys Lys Lys Leu Glu
        115                 120                 125

Gly His Pro Asp Gln Val Met Thr Leu Glu Val Trp Glu Lys Pro Trp
    130                 135                 140

Glu Asp Phe Leu Gln Val Asn Ile Leu Glu Thr Lys Val Leu Ser Ser
145                 150                 155                 160

Val

<210> 4
<211> 509
<212> PRT
<213> Trichuris suis

<400> 4

Met Lys Thr Ala Ile Leu Ile Phe Leu Ala Thr Ile Leu Thr Leu Lys
1               5                   10                  15

Pro Lys Ala Lys Cys Ala Ala Glu Ser Gly Ala Arg Ser Val Cys Asn
            20                  25                  30

Thr Val Arg Arg Arg Ser Ala Gly Met Leu Arg Lys Asp Ser Gln Asp
        35                  40                  45

Ala Ser Asp Glu Lys Tyr Val Arg Gly Cys Tyr Phe Thr Asn Trp Ala
    50                  55                  60

Gln Tyr Arg Pro Gly Asn Gly Lys Tyr Asn Pro Glu His Tyr Gln Ala
65                  70                  75                  80

Asn Leu Cys Glu Tyr Ile Phe Tyr Ala Phe Ala Lys Leu Asn Asp Asp

85                  90                  95

Phe Thr Val Asp Gln Phe Glu Trp Asn Asp Ile Asp Val Leu Tyr Pro
            100                 105                 110

Gly Val Met Lys Gln Lys Ser Ser Gln Pro Asp Leu Lys Val Leu Leu
        115                 120                 125

Ser Leu Gly Gly Trp Asn Ala Gly Thr Ala Thr Phe Lys Lys Met Ala
    130                 135                 140

Ala Thr Tyr Ser Asn Arg Ala Lys Phe Ile Ser Ser Leu Val Ser Phe
145                 150                 155                 160

Leu Gln Gln Asn Lys Phe Asp Gly Phe Asp Leu Asp Trp Glu Tyr Pro
                165                 170                 175

Glu Ser Ser Asp Lys Glu Asn Tyr Leu Leu Leu Cys Gln Glu Ile Leu
            180                 185                 190

Ala Lys Phe Glu Glu Val Ala Lys Cys Thr Ser Thr Ser Arg Leu Leu
        195                 200                 205

Phe Thr Ala Ala Val Ser Ala Asn Pro Lys Thr Val Asp Ala Gly Tyr
    210                 215                 220

Asp Val Pro Ala Leu Ala Lys Val Leu Asp Phe Val Asn Leu Met Cys
225                 230                 235                 240

Tyr Asp Phe His Gly Ala Trp Glu Thr Gln Thr Gly Ile Asn Ser Pro
                245                 250                 255

Leu Tyr Ser Arg Lys Glu Asp Ser Ser Glu Phe Lys Met Trp Asn Val
            260                 265                 270

Glu Gln Ser Ser Lys Tyr Trp Ser Asp Lys Gly Met Pro Lys Lys Gln
        275                 280                 285

Ile Ile Ile Gly Leu Pro Thr Tyr Gly Arg Gly Trp Thr Leu Ser Asp
    290                 295                 300

Ala Ser Lys Thr Asp Ile Gly Ala Pro Ala Gln Gly Ser Ser Thr Ala
305                 310                 315                 320

Thr Glu Tyr Leu Arg Glu Ala Gly Val Ile Ser Tyr Tyr Glu Val Cys
                325                 330                 335

Gln Lys Leu Ser Ser Gly Ala Lys Arg Val Trp Asp Asp Glu Ser Lys
            340                 345                 350

Thr Pro Tyr Leu Val Gln Gly Asn Gln Trp Phe Ser Tyr Asp Asp Val
        355                 360                 365

Glu Ser Met Lys Ala Lys Ile Asn Trp Ile Lys Gln Glu Asn Tyr Gly
    370                 375                 380

Gly Ala Phe Val Trp Thr Leu Asp Tyr Asp Asp Phe Leu Gly Ser Phe
385                 390                 395                 400

Cys Thr Glu His Asn Gly Lys Lys Tyr Pro Leu Ile Ser Leu Met Gln
                405                 410                 415

Glu Ile Leu Gly Gly Gly Tyr Val Pro Pro Ser Thr Glu Ser Thr Thr
            420                 425                 430

Ser Gln Val Thr Thr Thr Pro Ser Thr Thr Thr Ser Thr Thr Ser Pro
        435                 440                 445

Ala Gly Ala Phe Gln Cys Pro Ser Asp Gly Leu Phe Pro Asp Pro Glu
    450                 455                 460

Ser Cys Ser Asn Phe Tyr Gln Cys Ala Gly Gly Thr Ala Tyr Lys Met
465                 470                 475                 480

Lys Cys Pro Thr Gly Leu Met Phe Asn Pro Lys Thr Ser Thr Cys Asp
                485                 490                 495

Tyr Pro Ser Asn Val Asp Cys Gln Glu Lys Thr Ile Thr
            500                 505

<210>  5
<211>  298
<212>  PRT
<213>  Homo sapiens

<400>  5
   Met Ser Asp Ile Glu Glu Val Val Glu Glu Tyr Glu Glu Glu Glu Gln
   1               5                   10                  15
   Glu Glu Ala Ala Val Glu Glu Glu Glu Asp Trp Arg Glu Asp Glu Asp
               20                  25                  30
   Glu Gln Glu Glu Ala Ala Glu Glu Asp Ala Glu Ala Glu Ala Glu Thr
           35                  40                  45
   Glu Glu Thr Arg Ala Glu Glu Asp Glu Glu Glu Glu Glu Ala Lys Glu
       50                  55                  60
   Ala Glu Asp Gly Pro Met Glu Glu Ser Lys Pro Lys Pro Arg Ser Phe
   65                  70                  75                  80
   Met Pro Asn Leu Val Pro Pro Lys Ile Pro Asp Gly Glu Arg Val Asp
                   85                  90                  95
   Phe Asp Asp Ile His Arg Lys Arg Met Glu Lys Asp Leu Asn Glu Leu
               100                 105                 110
   Gln Ala Leu Ile Glu Ala His Phe Glu Asn Arg Lys Lys Glu Glu Glu
           115                 120                 125
   Glu Leu Val Ser Leu Lys Asp Arg Ile Glu Arg Arg Arg Ala Glu Arg
       130                 135                 140
   Ala Glu Gln Gln Arg Ile Arg Asn Glu Arg Glu Lys Glu Arg Gln Asn
   145                 150                 155                 160
   Arg Leu Ala Glu Glu Arg Ala Arg Arg Glu Glu Glu Glu Asn Arg Arg
                   165                 170                 175
   Lys Ala Glu Asp Glu Ala Arg Lys Lys Lys Ala Leu Ser Asn Met Met
               180                 185                 190
   His Phe Gly Gly Tyr Ile Gln Lys Gln Ala Gln Thr Glu Arg Lys Ser
           195                 200                 205
   Gly Lys Arg Gln Thr Glu Arg Glu Lys Lys Lys Lys Ile Leu Ala Glu
       210                 215                 220
   Arg Arg Lys Val Leu Ala Ile Asp His Leu Asn Glu Asp Gln Leu Arg
   225                 230                 235                 240
   Glu Lys Ala Lys Glu Leu Trp Gln Ser Ile Tyr Asn Leu Glu Ala Glu
                   245                 250                 255
   Lys Phe Asp Leu Gln Glu Lys Phe Lys Gln Gln Lys Tyr Glu Ile Asn
               260                 265                 270
   Val Leu Arg Asn Arg Ile Asn Asp Asn Gln Lys Val Ser Lys Thr Arg
           275                 280                 285
   Gly Lys Ala Lys Val Thr Gly Arg Trp Lys
       290                 295

<210>  6
<211>  210
<212>  PRT
<213>  Homo sapiens

<400>  6